(19) Europäisches Patentamt
European Patent Office
Office européen des brevets

(11) Veröffentlichungsnummer: **0 078 986**
**A1**

# (12) EUROPÄISCHE PATENTANMELDUNG

(21) Anmeldenummer: 82109937.1

(22) Anmeldetag: 27.10.82

(51) Int. Cl.³: C 07 D 491/04
C 12 P 17/18, A 01 N 43/90
//(C07D491/04, 239/00, 231/00, C12P17/18, C12R1/045)

(30) Priorität: 05.11.81 DE 3143972

(43) Veröffentlichungstag der Anmeldung: 18.05.83 Patentblatt 83/20

(84) Benannte Vertragsstaaten: AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Bischoff, Erwin, Dr.
Pahlkestrasse 73
D-5600 Wuppertal 1(DE)

(72) Erfinder: von Hugo, Hasso, Dr.
Am Eckbusch 67
D-5600 Wuppertal 1(DE)

(72) Erfinder: Klein, Udo, Dr.
Am Eckbusch 55B
D-5600 Wuppertal 1(DE)

(72) Erfinder: Reinecke, Paul, Dr.
Lessingstrasse 11
D-5090 Leverkusen 3(DE)

(72) Erfinder: Schedel, Michael, Dr.
Gellertweg 37
D-5600 Wuppertal 1(DE)

(72) Erfinder: Schmidt, Robert R., Dr.
Im Waldwinkel 110
D-5060 Bergisch Gladbach 2(DE)

(72) Erfinder: Zoebelein, Gerhard, Dr.
Am Domblick 46
D-5090 Leverkusen 3(DE)

## (54) Herbizide, insektizide und fungizide Mittel.

(57) Die Erfindung betrifft die Verwendung von Formycin A und B als herbizide, insektizide und fungizide Mittel, sowie die Herstellung von Formycin A durch die Fermentation des Actinoplanes Stammes SE 165.

EP 0 078 986 A1

Croydon Printing Company Ltd.

BAYER AKTIENGESELLSCHAFT  5090 Leverkusen, Bayerwerk

Zentralbereich
Patente, Marken und Lizenzen S/Kü/bo/c

IIb

# Herbizide, insektizide und fungizide Mittel

Die vorliegende Erfindung betrifft die neue Verwendung der bekannten Nucleoside Formycin A und Formycin B als Herbizide, Insektizide und Fungizide sowie ein neues Verfahren zur Herstellung von Formycin A.

Die Verwendung der Nucleoside Formycin A (teilweise auch Formycin genannt) und Formycin B als antimikrobielle Wirkstoffe ist bereits bekannt (vgl. Tetrahedron Letters 6, 597 - 602 (1966), Japanische Patentanmeldung 66 - 057296, J. Antibiotics, Ser. A 17 (3), 96 - 99 (1964) und J. Antibiotics Ser. A 19 (6), 268 - 287 (1966).

Es wurde nun gefunden, daß Formycin A und B der Formeln I und II starke herbizide und insektizide und fungizide Eigenschaften aufweisen.

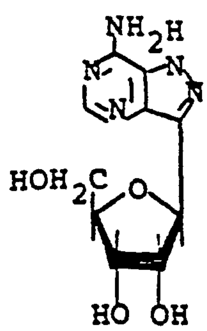

I (Formycin A)

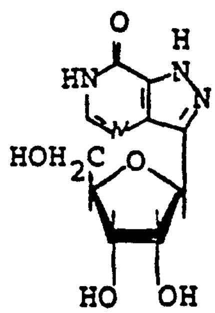

II (Formycin B)

Die neuen Eigenschaften der Formycine A und B sind im Hinblick auf den Stand der Technik überraschend und stellen in unerwarteter Weise eine Bereicherung der Möglichkeiten auf dem Pflanzenschutzgebiet dar.

Weiterhin wurde gefunden, daß man das erfindungsgemäß verwendbare Formycin A erhält, wenn man Mikroorganismen der Ordnung Actinomycetales in einem Nährmedium, welches assimilierbare Kohlenstoff- und Stickstoffquellen sowie Mineralsalze enthält unter aeroben Bedingungen kultiviert und die Verbindung isoliert.

Es war überraschend, daß durch die Fermentation von Mikroorganismen der Ordnung Actinomycetales Formycin A, welches frei von Formycin B ist, in vorteilhafter Weise erhalten werden kann.

Zur Gewinnung von Formycin A eignen sich innerhalb der Ordnung Actinomycetales besonders Vertreter der Familie Actinoplanaceae, und hier hauptsächlich solche aus der Gattung Actinoplanes. Das erfindungsgemäße Verfahren kann besonders gut mit Mikroorganismen der Art Actinoplanes durchgeführt werden. Als Beispiel sei Actinoplanes Stamm SE 165 (einschließlich seiner Varianten und Mutanten) genannt. Ein Muster dieses Stammes wurde unter der Nummer ATCC 31957 am 14. September 1981 bei der American Type Culture Cellection, Rockville, Maryland, U.S.A. hinterlegt.

Bakterienstämme, die zur Durchführung des erfindungsgemäßen Verfahrens geeignet sind, können aus Bodenproben

terrestrischen oder marinen Ursprungs durch die üblicherweise zu diesem Zweck angewandten Methoden isoliert werden. Der bevorzugte Actinoplanes Stamm SE 165 beispielsweise konnte aus einer Bodenprobe angereichert werden, die im März 1971 im Jordan-Tal, Israel, genommen wurde.

Der Stamm wird seit dieser Zeit auf geeigneten festen Nährböden (z.B. auf HPG-Agar: 3 g Hefeextrakt, 6 g Pepton, 10 g Glucose, 8 g NaCl, 20 g Agar, 1000 ml entmineralisiertes Wasser, pH-Wert auf 7,0 eingestellt) in Kultur gehalten.

Actinoplanes spec. Stamm SE 165 hat folgende Merkmale:

| | |
|---|---|
| <u>Mycel:</u> | Substratmycelhyphen verzweigt, mit geradem, geschlängeltem bis korkenzieherartigem Verlauf. Hyphen 0,5 bis 1,5 µm dick, dicke Hyphen oft stark septiert. |
| <u>Sporangien:</u> | unregelmäßig kugelförmig, im ausgereiften Zustand oft buckelig bis grob gelappt. Durchmesser 10 bis 22 µm; Sporangiophoren 1,8 bis 2,2 µm dick. Sporen in Reihen gebildet, diese aber im Sporangium nicht einheitlich ausgerichtet. |
| <u>Sporen:</u> | annährend kuglige Form ("subglobose") Durchmesser im ungequollenen Zustand 1,0 bis 1,5 µm; Sporen schwärmen nach 15 Min. |

in Leitungswasser und destilliertem Wasser aus den Sporangien aus, sie sind begeißelt, zeigen lebhafte Schwimmbewegungen mit häufig wechselnden Richtungsänderungen.

Wachstum auf verschiedenen Nährmedien:

| | | |
|---|---|---|
| Hefe-Stärke | W | gut |
| Agar | SM | ockerorange |
| | "LM" | ++, reifartiger Überzug |
| | SP | gelblich |
| Haferflocken- | W | gut bis sehr gut |
| Hefe-Agar | SM | orange |
| | "LM" | gering |
| | SP | schwach ockerorange |
| Casamino-Pepton | W | gut |
| Czapek-Agar | SM | orangebraun |
| | "LM" | ++, glänzender Überzug |
| | SP | dunkelbraun |
| Erdextrakt- | W | mäßig |
| Hefe-Agar | SM | ocker |
| | "LM" | ++, matter Überzug in konzentrischen Kreisen |
| | SP | - |
| Erd-Glucose- | W | mäßig bis gut |
| Hefe-Agar | Sm | ocker |
| | "LM" | ++, grauer, matter Überzug |
| | SP | - |
| Magermilch- | W | gut bis sehr gut |
| Agar | SM | orange |

| | | |
|---|---|---|
| | "LM" | +, reifartiger, granulierter Überzug |
| | SP | ockerorange |
| | Milch | wird peptonisiert |
| Nutrient-Agar | W | mäßig bis gering |
| | SM | orange |
| | LM | -, matt schimmernde Oberfläche |
| | SP | ockerbraun |
| Tyrosin-Agar | W | mäßig |
| | SM | orange |
| | "LM" | +, glänzender Überzug |
| | SP | - |
| Malz-Hefe-Glucose-Agar | W | gut bis sehr gut |
| | SM | orange |
| | LM | ++, an trockenen Stellen |
| | SP | ocker |
| Glucose-Asparagin-Agar | W | gut |
| | SM | ockerorange |
| | "LM" | ++, glänzender Überzug |
| | SP | - |
| Glycerin-Aspargin-Agar | W | gut |
| | "LM" | +, stellenweise reifartiger Überzug |
| | SP | ocker |
| $NO_3$-Agar | W | mäßig bis gut |
| | SM | orangebräunlich |
| | "LM" | - |
| | SP | ockerbraun |
| | Keine | Nitrit-Bildung aus Nitrat |
| Czapek-Agar (Cz) | W | mäßig bis gut |
| | SM | orange |
| | "LM" | +, schwacher Überzug |
| | SP | - |

Physiologische Eigenschaften:

.Wachstum bei

| | |
|---|---|
| 15°C | ++ |
| 20 | ++ |
| 29 | + |
| 37 | - |
| 42 | - |
| 50 | - |

.Kochsalzverträglichkeit: 1%: ++; 2%: +; >3%:-

Melaninbildung: -

Nitratreduktion: -

Gelatineverflüssigung: ±

Milchpeptonisierung: +

Tyrosinauflösung: -

.Stärke-Hydrolyse: +

.Verwertbarkeit verschiedener C-Quellen:

| | |
|---|---|
| L-Arabinose: | ++ |
| Raffinose: | - |
| D-Fructose; | ++ |
| D-Xylose: | ++ |
| D-Glucose: | ++ |
| L-Rhamnose: | ++ |
| i-Inosit: | + |
| Saccharose: | ++ |
| D-Mannit: | ++ |

.Verwertbarkeit von Cellulose: -

Für die Kultivierung kommen alle üblichen Kohlenstoffquellen infrage.

Als Beispiele seien Stärke, Melasse, Molkepulver, Dextrin und Zucker, wie Saccharose, Maltose, Glucose, Lactose, Sorbit und Glycerin genannt. Als Stickstoffquellen kommen alle üblichen organischen und anorganischen Stickstoffquellen infrage. Als Beispiel seien Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt sowie Ammoniumsalze und Nitrate, z.B. $NH_4Cl$, $(NH_4)_2SO_4$, $NaNO_3$ und $KNO_3$ aufgeführt. Die Mineralsalze, welche im Nährmedium enthalten sein sollen, liefern z.B. folgende Ionen:

$Mg^{++}$, $Na^{+}$, $K^{+}$, $Ca^{++}$, $NH_4^{+}$, $Cl^{-}$, $SO_4^{--}$, $PO_4^{---}$ und $NO_3^{-}$ sowie Ionen der üblichen Spurenelemente, wie Cu, Fe, Mn, Mo, Zn, Co, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen.

Bei der Durchführung des Verfahrens kann es günstig sein, zu Beginn der Kultivierung nur relativ geringe Konzentrationen der löslichen Nährlösungsbestandteile zu verwenden und dann im Laufe der Kultivierung diese Bestandteile in Form steriler, relativ konzentrierter Lösungen dem Kulturansatz fraktioniert zufüttern. Der pH-Wert der wachsenden Kulturen sollte zwischen etwa 5,5 und etwa 9,5 gehalten werden. Ein zu starker pH-Abfall in den sauren Bereich kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säure, z.B. $H_2SO_4$, oder sterile Lauge, z.B. NaOH in Abständen in die Kulturlösung eingespritzt wird.

Es ist zweckmäßig sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 10 und etwa 40°C liegen. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden Verbindung im allgemeinen ihr Maximum zwischen dem 1. und 12., vorzugsweise zwischen dem 4. bis 6.Tag nach Züchtungsbeginn erreicht.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Vorrichtungen können z.B. die Dampf- als auch die Trockensterilisation verwendet werden.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöl, Polyoxyethylen- bzw. Polyoxypropylenverbindungen zugesetzt werden. Schaum kann auch mit Hilfe der üblichen mechanischen Vorrichtungen (welche z.B. Zentrifugalkräfte benutzen) gedämpft oder beseitigt werden.

Das Formycin A kann aus dem Kulturmedium nach allgemein üblichen physikalisch-chemischen Methoden isoliert werden. Die Isolierung kann z.B. gemäß den üblichen Extraktionsverfahren, Fällungsverfahren und/oder Chromatographieverfahren erfolgen. Das isolierte Nucleosid Formycin A kann mit Hilfe der genannten Methoden auch feingereinigt werden. Für viele Fälle ist jedoch eine Feinreinigung nicht erforderlich, da die gegebenenfalls vorhandenen Verunreinigungen die Wirksamkeit des Nucleosids Formycin A als Herbizid oder Insektizid oder Fungizid nicht nachteilig beeinflussen. Um bei den o.a. Isolierungs- und Reinigungsmethoden die Fraktionen herauszufinden, in welchen das erfindungsgemäße Nucleosid Formycin A in höchster Konzentration bzw. Reinheit vorliegt, können die üblichen physikalisch-chemischen Methoden, z.B. Messen der UV-Absorption, der $R_f$-Werte oder vorzugsweise die Untersuchung der herbiziden oder insektiziden oder fungiziden Aktivität herangezogen werden.

Die Isolierung und Reinigung des erfindungsgemäßen Wirkstoffs kann z.B. im Falle, daß ein flüssiges wässriges Nährmedium verwendet wird, wie folgt vorgenommen werden: Nach seiner Anreicherung im Kulturüberstand werden Kulturfiltrat und Mycel mit üblichen Methoden separiert (z.B. Zentrifugation).

Aus dem Kulturfiltrat kann das Formycin A mit Hilfe von üblichen Extraktionsverfahren, Fällungsverfahren und/oder Chromatographieverfahren isoliert und gegebenenfalls gereinigt werden. Die Chromatographie kann in Form der Säulenchromatographie durchgeführt werden. Als Adsorptionsmittel können die üblichen anorganischen oder organischen Adsorptionsmittel eingesetzt werden, wie z.B. Aluminiumoxid, Kieselgel, Magnesiumsilikat, Aktivkohle, Cellulose, Cellulose-Derivate, Kunstharze wie Polyamide, Derivate von Polyamiden, z.B. acetyliertes Polyamid oder Dextrangele. Als Laufmittel können die verschiedensten Lösungsmittel oder Lösungsmittelgemische verwendet werden, in welchen der erfindungsgemäße Wirkstoff löslich ist. Bevorzugt wird Wasser oder ein Gemisch aus Wasser und Methanol oder aus Wasser und Ethanol eingesetzt.

Bevorzugt werden zur Isolierung von Formycin A Chromatographie-Verfahren verwendet, z.B. unspezifische Adsorption an hydrophobe Sorbentien oder andererseits Ionenaustauschchromatographie. Dieses sind die von der Reinigung wasserlöslicher basischer Naturstoffe her bekannten Methoden.

Für die technische Herstellung von Formycin A bevorzugt man die Gewinnung durch Adsorption und anschließende Desorption an einem hydrophoben Trägerharz (z.B. Lewapol). Die Desorption kann z.B. mit kurzkettigen aliphatischen Alkoholen durchgeführt werden, bevorzugt Methanol oder Ethanol. Das Desorptionsmittel sollte mit Wasser mischbar sein, da die Löslichkeit des Nucleosids in wasserfreien Alkoholen gering ist.

Weiterhin kann eine Ionenaustauschchromatographie durchgeführt werden, vorzugsweise an Ionenaustauschern vom Phosphorsäuretyp (z.B. Phosphocellulose) oder vom Sulfonsäuretyp (z.B. Dowex$^{(R)}$50-Typen[2]), aber auch schwächer saure Austauscher vom Carbonsäuretyp (wie z.B. Lewatit$^{(R)}$CNP/LF oder Amberlite IRC-50) können eingesetzt werden. Der Ionenaustausch kann mit Hilfe der Gradientenelution durchgeführt werden, wobei sowohl reine pH-Gradienten, reine Salzgradienten, als auch gemischte pH/Salz-Gradienten erfolgreich eingesetzt werden können. Der Ionenaustausch kann auch im "batch"-Verfahren durchgeführt werden, wobei zur Desorption vorzugsweise höhere Salzkonzentrationen in Wasser verwendet werden (z.B. 1 M Ammoniumformiat in Wasser),

aber auch durch Erhöhung des pH-Wertes (z.B. auf pH 10) kann die Desorption erreicht werden.

Eine derart vorgereinigte Nucleosid-Fraktion kann wiederum mit den üblichen Methoden gereinigt werden. Bevorzugt kann hier die Geldiffusionschromatographie eingesetzt werden. Die Chromatographie, beispielsweise an Sephadex G-10, verläuft erfolgreich, die Isolierung des Nucleosids Formycin A kann aber auch an modifizierten Dextrangelen durchgeführt werden (z.B. Sephadex LH-20). Vorzugsweise wird hier in Wasser, kurzkettigen aliphatischen Alkoholen, wie z.B. Methanol oder Gemischen aus diesen Alkoholen mit Wasser chromatographiert.

Die Reinsubstanz des erfindungsgemäßen Nucleosids Formycin A kann aus einer solchen Fraktion mit den üblichen biochemischen Methoden gewonnen werden. Von den bereits genannten Adsorptionsmitteln kann neben anderen Kieselgel erfolgreich eingesetzt werden. Als Flußmittel kommt z.B. Chloroform-Methanol (so etwa 4/1 Volumenteile) mit Erfolg zur Anwendung.

Aus seinen Lösungen kann das Nucleosid Formycin A nach den üblichen Methoden, z.B. Verdampfen des Lösungsmittels, Gefriertrocknung usw. erhalten werden.

Wie bereits erwähnt sind Formycin A und B herbizid, insektizid und fungizid (vorzugsweise Formycin A) wirksam. Wegen seines breiteren Wirkungsspektrums ist Formycin A bevorzugt.

Die erfindungsgemäß verwendbaren Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dioctyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactua, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria,

Eleocharis, Scirpus, Paspalum, Ischaemum, Sphenoclea, Dactyloctenium, Agrostis, Alopecurus, Apera.

Monokotyle Kulturen der Gattungen: Oryza, Zea Triticum, Hordeum, Avena, Secale, Sorghum, Panicum, Saccharum, Ananas, Asparagus, Allium.

Die Verwendung der Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjähringen Kulturen eingesetzt werden.

Die Wirkstoffe zeigen insbesondere neben einer sehr guten Wirkung gegen grasartige Unkräuter auch eine gute herbizide Wirkung bei breitblättrigen Unkräutern. Formycin A und B können auch zur selektiven Bekämpfung von Unkräutern in verschiedenen Kulturgattungen eingesetzt werden.

Formycin A und B können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die Wirkstoffe können sowohl vor als auch nach dem Auflaufen der Pflanzen appliziert werden. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 20 kg Wirkstoff pro ha, vorzugsweise zwischen 0,1 und 10 kg/ha, insbesondere 0,1 bis 5 kg/ha.

Die erfindungsgemäß verwendbaren Nucleoside Formycin A und Formycin B weisen eine starke insektizide Wirkung auf. Sie können deshalb auch zur Bekämpfung von tierischen Schädlingen eingesetzt werden.

Die erfindungsgemäß verwendbaren Wirkstoffe eignen sich hierbei bei günstiger Warmblütertoxizität insbesondere zur Bekämpfung von Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören beispielsweise:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.,

Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinia spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana,

Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Fannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.
Aus der Ordnung der Arachnida z.B. Scorpio maurus,

Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Formycine A und B, insbesondere Formycin A können auch vorteilhaft zur Bekämpfung von unerwünschten Mikroorganismen insbesondere unerwünschten Pilzen eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können Formycin A und B (besonders Formycin A) z.B. zur Bekämpfung von Erysiphe-Arten, wie z.B. gegen den Erreger des Gurkenmehltaues (Erysiphe cichoracearum) eingesetzt werden sowie auch zur Bekämpfung von Phytophthora-Arten, wie z.B.

gegen den Erreger der Braunfäule der Tomate (Phythophtora infestans) und gegen den Erreger des Weizenbraunrostes (Puccinia recondita). Insbesondere ist Formycin A wirksam gegen Pyricularia oryzae an Reis.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase,wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können zur Wachstumsregulierung bzw. als Herbizide und als Mittel gegen tierische Schädlinge in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielswieise Phosphorsäureester, Carbamate, Carbonsäureester, chlorierte Kohlenwasserstoffe, Phenylharnstoffe, durch Mikroorganismen hergestellte Stoffe u.a.

Die Wirkstoffe können ferner in handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann z.B. bei der Verwendung als Insektizide von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Die Formycine A und B, insbesondere Formycin A können zur Bekämpfung von Pilzen ebenfalls in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Der Wirkstoff kann als solcher in Form seiner Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kg Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Formycine A und B können in den beschriebenen Anwendungsarten entweder einzeln oder als Gemische untereinander verwendet werden.

Die Wirksamkeit der erfindungsgemäß verwendbaren Formycine A und B soll anhand der folgenden Beispiele erläutert werden. Beispiel A - C beziehen sich auf die herbizide Wirksamkeit von Formycin A und B. Beispiele D - F beziehen sich auf die insektizide Wirksamkeit von Formycin A und B und Beispiel G illustriert die fungizide Wirksamkeit von Formycin A.

Beispiel A

post-emergence-Test

In Schalen, die mit Vermiculite gefüllt sind, werden Samen von Testpflanzen ausgelegt. Die Schalen werden dann mit einer Hoagland-Nährlösung gegossen, bis die Pflanzen eine Größe von 5 bis 10 cm erreicht haben.

Dann werden die Pflanzen mit einer Wirkstoffzubereitung gespritzt.

Nach 2 Wochen wird der Schädigungsgrad der Pflanzen bonitriert in % Schädigung im Vergleich zur Entwicklung der unbehandelten Kontrolle.

Die Präparate gemäß den obigen Beispielen zeigen eine hohe Wirksamkeit, wie aus der folgenden Tabelle ersichtlich ist.

0078986

**Beispiel A**

post-emergence-Test (Vermiculite)

| Wirkstoff | Aufwand menge kg/ha | Chenopodium album | Lepidium sativum | Stellaria media | Portulaca oleracea | Echinochloa crus galli | Poa annua |
|---|---|---|---|---|---|---|---|
| Formycin A | 4 | 100 | 100 | 95 | 100 | 80 | 80 |
| Formycin B | 4 | 95 | 100 | 90 | 50 | 90 | 95 |

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Wirkstoffe, Aufwandmengen und Resultate gehen aus der nachfolgenden Tabelle hervor:

**Beispiel B**

pre-emergence-Test (Boden)

| Wirkstoff | Aufwand menge kg/ha | Chenopodium album | Lepidium sativum | Stellaria media | Portulaca oleracea | Echinochloa crus galli | Poa annua |
|---|---|---|---|---|---|---|---|
| Formycin A | 4 | 40 | 100 | 98 | 85 | 0 | 50 |
| Formycin B | 4 | 30 | 98 | 95 | 65 | 60 | 75 |

Beispiel C

pre-emergence-Test

In Schalen, die mit Vermiculite gefüllt sind, werden Samen von Testpflanzen ausgelegt. Die Schalen werden dann mit einer Hoagland-Nährlösung gegossen, der die erfindungsgemäßen Wirkstoffe zugesetzt sind.

Nach 2 Wochen wird der Schädigungsgrad der Pflanzen bonitiert in % Schädigung im Vergleich zur Entwicklung der unbehandelten Kontrolle.

Die Präparate gemäß den obigen Beispielen zeigen eine hohe Wirksamkeit, wie aus der folgenden Tabelle ersichtlich ist:

**Beispiel C**

pre-emergence-Test (Vermiculite)

| Wirkstoff | Aufwand menge kg/ha | Chenopodium album | Lepidium sativum | Stellaria media | Portulaca oleracea | Echinochloa crus galli | Poa annua |
|---|---|---|---|---|---|---|---|
| Formycin A | 4 | 90 | 100 | 100 | 90 | 90 | 85 |
| Formycin B | 4 | 100 | 100 | 100 | 90 | 98 | 98 |

Beispiel D

Aedes-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Man füllt die wäßrigen Wirkstoffzubereitungen in Gläser und setzt anschließend etwa 20 Mückenlarven (Aedes aegypti) in jedes Glas ein.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Ergebnis:

| Wirkstoffe | Konzentration (%) | (%) Abtötung nach 14 Tagen |
|---|---|---|
| Formycin A | 0,001 | 100 |
| | 0,0001 | 87 |
| Formycin B | 0,001 | 87 |
| | 0,0001 | 87 |

Beispiel E

Plutella-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebene Menge Emulgator und verdünnt das Konzentrat mit Wasser auf die gewünschte Konzentration.

Kohlpflanzen (Brassica oleracea) werden durch Sprühen mit der Wirkstoffzubereitung der gewünschten Konzentration behandelt. Nach Antrocknen der Spritzbrühe wird von der behandelten Pflanze ein Blatt entnommen, in eine Plastikdose gelegt und mit Raupen der Kohlschabe (Plutella maculipennis) besetzt. Nach 2 und 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Ergebnisse:

| Wirkstoffe | Konzentration (%) | % Abtötung nach 14 Tagen |
|---|---|---|
| Formycin A | 0,1 | 80 |
| | 0,01 | 70 |
| Formycin B | 0,1 | 100 |
| | 0,01 | 88 |

Beispiel F

Phaedon-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Kohlpflanzen (Brassica oleracea) werden durch Sprühen mit der Wirkstoffzubereitung der gewünschten Konzentration behandelt. Nach Antrocknen der Spritzbrühe wird von der behandelten Pflanze ein Blatt entnommen, in eine Plastikdose gelegt und mit Larven des Meerrettichkäfers (Phaedon cochleariae) besetzt. Nach 2 und 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Ergebnisse:

| Wirkstoffe | Konzentration % | % Abtötung nach 14 Tagen |
|---|---|---|
| Formycin A | 0,1 | 100 |
| | 0,01 | 50 |
| Formycin B | 0,1 | 100 |
| | 0,01 | 80 |

Beispiel G

Pyricularia-Test (Reis) / protektiv

Lösungsmittel: 50 Gew.-Teile Wasser
Emulgator: 0,15 Gew.-Teile Alkylarylpolyglycolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Formycin A ergab bei einer Wirkstoffkonzentration von 0,02 % eine völlige Befallsfreiheit (Krankheitsbefall = 0 %, bezogen auf die unbehandelte Kontrolle).

Das erfindungsgemäße Verfahren zur Herstellung von Formymicin A soll anhand der folgenden Beispiele erläutert werden:

Beispiel H

1. Fermentation des Actinoplanaceenstammes SE 165

Auf Schrägagar der Zusammensetzung

| | | |
|---|---|---|
| Maisstärke | | 2,0 % |
| D(+)-Glucose | | 1,0 % |
| NZ-Amine (Seffield Chem.) | | 0,5 % |
| Hefeautolysat | | 1,0 % |
| $CaCO_3$, p.a. | | 0,4 % |
| Agar | | 2,0 % |
| Wasser | ad | 100 % |

gewachsenes Myzel des Stammes Actinoplanes SE 165 wird auf je 140 ml steriler, i 1 ltr.-Erlenmeyerkolben befindlicher Nährlösung (im folgenden "Nährlösung A" genannt) der Zusammensetzung

| | | |
|---|---|---|
| Sojamehl entfettet | | 3,0 % |
| Glycerin, reinst | | 3,0 % |
| $CaCO_3$, p.a. | | 0,2 % |
| Wasser | ad | 100 % |

und Hydroxylgruppen enthaltendes neutrales Polyol, z.B. Niax LHT 67 (Warenzeichen der Union Carbide Belg. N.V.), 1 Tropfen/140 ml Nährlösung zur Entschäumung überimpft. Die Kolben werden 4 Tage bei 28°C auf der Rundschüttelmaschine inkubiert. Je 20 l Nährlösung A und 60 ml Hydroxylgruppenhaltiges neutrales Polyol (z.B. Niax Polyol LHT) werden in Fermentern mit Rührwerk und Belüftungseinrichtung abgefüllt und bei 121°C sterilisiert. Nach Abkühlung der Lösungen werden die Fermenter mit je 900 ml von 4 Tage in Nährlösung A gewachsenen Schüttelkulturen des Actinoplanes-Stammes SE 165 beimpft, mit etwa 20 l Luft/min. bei etwa 300 U/min des Rührwerks belüftet und bei einer Temperatur von 28°C gehalten.

Je 200 l Nährlösung A und 600 ml Hydroxylgruppenhaltiges neutrales Polyol je 200 l Nährlösung werden in Fermentern mit Rührwerk und Belüftungseinrichtung abgefüllt und bei 121°C sterilisiert. Nach Abkühlung der Lösungen werden die Fermenter mit je 20 l von 3 Tage in Nährlösung A gewachsenen Vorfermentern mit dem Stamm SE 165 beimpft und mit etwa 200 l Luft/min. belüftet und bei einer Temperatur von 28°C gehalten.

Nach 4-tägiger Fermentation des Stammes werden Proben der Kulturen zentrifugiert. Die klare überstehende Lösung wird in üblicher Weise im Agarplattenlochtest gegen Pyricularia oryzae getestet. Das in der Kulturlösung befindliche erfindungsgemäße Wirkstoffgemisch verursacht Wachstumshemmzonen von etwa 20 mm Durchmesser. Die Isolierung des erfindungsgemäßen Wirkstoffes Formycin A aus den Kulturbrühen geschieht wie im folgendem Beispiel I beschrieben.

Dieser Agarplattenlochtest ist auch sehr gut geeignet in einem Routineverfahren Produktionsstämme in einfacher Weise aufzufinden.

Beispiel I

Bei einer Fermentation nach Beispiel H werden nach dem Abzentrifugieren des Mycels 250 l Kulturüberstand gewonnen. Der Kulturüberstand wird mit 20 l/h auf eine Lewapol-Säule (30x50 cm) aufgetragen. Nach Vordesorption mit 300 l Wasser und 300 l wäßrigem 30 %igem Methanol wird die gesamte Aktivität mit 300 l Methanol desorbiert. Das Desorbat wird unter reduziertem Druck konzentriert, in Wasser zurückgelöst und lyophilisiert. Es werden 96 g eines ca. 5 %igen Rohproduktes erhalten.

30 g dieses Materials werden in 1 l Wasser gelöst und in einer Säule von 5 cm Ø an 600 g Lewatit CNP 80 ($H^+$-Form) adsorbiert. Nach Vordesorption mit 4 l Wasser wird mit 5 l wäßrigem 80 %igem Methanol desorbiert. Das Desorbat wird unter reduziertem Druck konzentriert. Nach Gefriertrocknung erhält man 3,05 g eines ca. 20-25 %igen Produktes.

3 g dieser Substanz werden in 30 ml 80 %igem wäßrigem Methanol gelöst und auf Sephadex LH 20 aufgetrennt (Säule: 5 cm Ø x 1 m Länge, Fließmittel: 80 %iges wäßriges Methanol, Fließrate: 120 ml/h, 20 ml-Fraktionen wurden genommen). Die Fraktionen 72-88 zeigen bei 278 nm eine starke Absorption und enthielten den überwiegenden Anteil der biologischen Aktivität. Nach

dem Gefriertrocknen werden 620 mg eines 60-80 %igen Materials erhalten.

100 mg dieses Produktes werden auf zwei präparierte Kieselgelplatten (Merck 5717) aufgetragen und mit Chloroform/Methanol/Wasser (80:20:2,5; V/V/V) entwickelt. Die "UV-aktive" Zone, die mit der biologischen Aktivität korreliert, wird ausgekratzt und mit 2x50 ml Methanol extrahiert, konzentriert und lyophilisiert. Danach ergaben sich 33 mg einer in der Dünnschichtchromatographie im 100 µg-Maßstab einheitlichen, farblosen Substanz.

Die Substanz konnte durch Vergleich der Infrarotspektren, der UV-Spektren bei neutralem, saurem und alkalischem pH-Wert und durch Vergleich der $R_f$-Werte in drei verschiedenen Laufmitteln (sowohl einzeln als auch im Gemisch) und des Mischschmelzpunktes von authentischem Formycin A und der isolierten Substanz als Formycin A identifiziert werden.

Folgende Warenzeichen wurden in der Beschreibung und den Beispielen aufgeführt:

LEWAPOL
LEWATIT (Lewatit CNP80/CNP/LF) Warenzeichen der Bayer AG, Leverkusen, Bundesrepublik Deutschland

DOWEX Warenzeichen der DOW Chemical Co., Midland, USA,

AMBERLITE (JRC-50) Warenzeichen der Rohm & Haas, USA,

SEPHADEX (LH 20/G10) Warenzeichen der Firma Pharmacia, Uppsala, Schweden

NIAX (Polyol L HT 67) Warenzeichen der Union Carbide N.V., Belgien

Patentansprüche

1. Herbizide, insektizide und fungizide Mittel, gekennzeichnet durch einen Gehalt an Formycin A und/oder Formycin B.

2. Verfahren zur Bekämpfung von Unkräutern, Insekten und Pilzen, dadurch gekennzeichnet, daß man Formycin A und/oder Formycin B auf die Unkräuter, Insekten und Pilze oder ihren Lebensraum einwirken läßt.

3. Verwendung von Formycin A und B oder von Schädlingsbekämpfungsmitteln, die Formycin A und/oder Formycin B enthalten zur Bekämpfung von Unkräutern, Insekten und Pilzen.

4. Herstellung von Formycin A und/oder Formycin B enthaltenden herbiziden, insektiziden und/oder fungiziden Mitteln, dadurch gekennzeichnet, daß man Formycin A und/oder Formycin B mit Streckmitteln und/oder oberflächenaktiven Mitteln vermischt.

5. Verfahren zur Herstellung von Formycin A, dadurch gekennzeichnet, daß man solche Mikroorganismen der Ordnung Actinomycetales, welche Formycin A erzeugen nach üblichen Methoden auswählt und in einem Nährmedium, welches assimilierbare C- und N-Quellen sowie Mineralsalze enthält unter aeroben Bedingungen kultiviert und Formycin A nach üblichen Methoden isoliert.

6. Verfahren gemäß Anspruch 5, dadurch gekennzeichnet, daß man als Mikroorganismen solche der Familie Actinoplanaceae einsetzt.

7. Verfahren gemäß Anspruch 6, dadurch gekennzeichnet, daß man als Mikroorganismen solche der Gattung Actinoplanes einsetzt.

8. Verfahren gemäß Anspruch 7, dadurch gekennzeichnet, daß man den Actinoplanes Stamm SE 165 oder seine Mutanten und Varianten einsetzt.

9. Formycin A erhalten nach dem Verfahren gemäß den Ansprüchen 5 bis 9.

10. Actinoplanes Stamm SE 165 und seine Mutanten und Varianten.

Europäisches Patentamt

# EUROPÄISCHER RECHERCHENBERICHT

0078986

Nummer der Anmeldung

EP 82 10 9937

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | THE JOURNAL OF ANTIBIOTICS, Band XXVII, Nr. 12, 1974, Seiten 909-916, Japan Antibiot. Res. Association, KOZO OCHI et al.: "Biosynthesis of formycin role of certain amino acids in formycin biosynthesis" * Insgesamt * | 1,2,5 | C 12 P 17/18<br>C 07 D 491/04<br>A 01 N 43/90 //<br>(C 07 D 491/04<br>C 07 D 239/00<br>C 07 D 231/00<br>C 12 P 17/18<br>C 12 R 1/045) |
| X | BIOLOGICAL ABSTRACTS, Band 57, 15. Juni 1974, Biological Abstract Service, Nr. 68687 N. TEZUKA et al.: "Effects of some chemicals on the multiplication of cauliflower mosaic virus" & ANN PHYTOPATHOL SOC JAP 39(3): 259-265, Illus. 1973 * Zusammenfassung * | 1-5 | RECHERCHIERTE SACHGEBIETE (Int. Cl. 3) |
| X | DERWENT JAPANESE PATENTS REPORT, Band 7, Nr. 19, 17. Juni 1968, Derwent Publications Ltd., London, G.B. "Agricultural and horticultural fungicide" & JP - A - 68 11294 (NIPPON NOYAKU CO., LTD.) 13.05.1968 * Zusammenfassung * | 1-4 | C 12 P 17/00<br>C 12 R 1/00<br>A 01 N |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 01-02-1983 | RAJIC M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03.82

Europäisches Patentamt

**EUROPÄISCHER RECHERCHENBERICHT**

0078986

Nummer der Anmeldung

EP 82 10 9937

| EINSCHLÄGIGE DOKUMENTE | | | Seite 2 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| D,X | DERWENT JAPANESE PATENTS REPORT, Band R, Nr. 6, 17. März 1970, Seite 18, Derwent Publications Ltd., London, G.B. "Agricultural tungicide containing formycin A or B and inorg cpds." & JP - A - 70 02597 (NIPPON KAYAKU K.K.) 28.01.1970 * Zusammenfassung * --- | 1-4 | |
| D,X | TETRAHEDRON LETTERS, Nr. 6, 1966, Seiten 597-602, Pergamon Press Ltd., Oxford, G.B. GUNJI KOYAMA et al.: "The structural studies of formycin and formycin B" * Insgesamt * ----- | 1,2,5 | |
| | | | RECHERCHIERTE SACHGEBIETE (Int. Cl. 3) |

Der vorliegende Recherchenbericht wurde für alle Patentansprüche erstellt.

| Recherchenort | Abschlußdatum der Recherche | Prüfer |
|---|---|---|
| DEN HAAG | 01-02-1983 | RAJIC M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument